# EUROPEAN PATENT APPLICATION

(11) **EP 4 173 623 A1**
(43) Date of publication of application: **03.05.2023**
(21) Application number: 21827874.5
(22) Date of filing: 25.06.2021
(51) Int. Cl.: A61K 31/4741, A61K 45/00, A61P 9/06

(54) **THERAPEUTIC AGENT FOR DRUG-INDUCED BRADYCARDIA AND BRADYARRHYTHMIA**

(30) Priority: 26.06.2020 JP 2020110975
(71) Applicant: OSAKA UNIVERSITY, Suita-shi Osaka 565-0871 (JP); Nissan Chemical Corporation, Tokyo 103-6119 (JP)
(72) Inventor: TAKASHIMA, Seiji, Suita-shi, Osaka 565-0871 (JP); ASANO, Yoshihiro, Suita-shi, Osaka 565-0871 (JP); YAMADA, Noriaki, Suita-shi, Osaka 565-0871 (JP); KONDO, Yoshiki, Shiraoka-shi, Saitama 349-0294 (JP); KAWAI, Yu, Shiraoka-shi, Saitama 349-0294 (JP)
(74) Representative: Hoffmann Eitle
(86) International application number: PCT/JP2021/024254
(87) International publication number: WO 2021/261598

(57) **Abstract**

There is provided a therapeutic agent for drug-induced bradycardia and bradyarrhythmia. A therapeutic agent for drug-induced bradycardia and bradyarrhythmia comprising, as an active ingredient, the following compound (I): 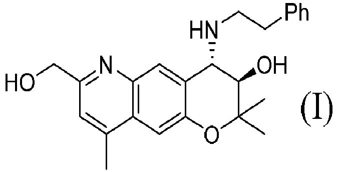 (wherein Ph is a phenyl group) or a pharmacologically acceptable salt of the compound.

## Description

### TECHNICAL FIELD

The present invention relates to a therapeutic agent for drug-induced bradycardia and bradyarrhythmia.

### BACKGROUND ART

Arrhythmias are conditions in which the heart rate or heart pulsation rhythm is irregular, and have various electrocardiographic abnormalities. Arrhythmia due to abnormal heart rate can be classified into tachyarrhythmia and bradyarrhythmia. The heart rate of humans at rest is generally about 50 beats/minute to 100 beats/minute, and bradycardia is a heart rate lower than that and tachycardia is a heart rate higher than that. Examples of bradyarrhythmias include sick sinus syndrome (SSS), atrioventricular block (A-V block), atrioventricular dissociation (A-V dissociation), and junctional rhythm.

Sick sinus syndrome is a disease in which the pulse is slowed mainly due to decline of sinus node functions, and as a result, dysfunction of the brain, heart, kidney, and the like is observed. Electrocardiographically, it is classified into three types: 1) sinus bradycardia (heart rate: 50 beats/minute or less), 2) sinus arrest or sinoatrial block, and 3) bradycardia tachycardia syndrome. Clinically, symptoms such as Adam-Stokes attacks, heart failure, and easy fatigability appear chronically. As a treatment method, in order to increase the number of spontaneous excitements of the sinus node, oral drugs such as an anticholinergic agent (atropine sulfate) and a β stimulant, or intravenous injection agents may be used, and if bradycardia is not improved with these administrations, or if symptoms worsen when drug administration is discontinued, a pacemaker may be used.

The sinus node, which is the beginning of electrical stimulation in the heart, is a tissue in which a part of the myocardium is specialized and has an acquired automatic ability, and specific proteins are expressed therewith. Among these, the most important function for the sinus node is that of a cell membrane protein group called a channel. Channels contribute to formation of potential gradients inside and outside cells by allowing ions to permeate through the cell membrane, and not just for the sinus node, are involved in the most important functions of the heart which are the propagation of electronic signals in all cardiomyocytes, and subsequent contraction and relaxation of the heart.

Drug-induced bradyarrhythmia refers to a condition in which the aforementioned bradyarrhythmia is induced by a drug administered for the purpose of treating a certain disease.

Regarding drug-induced bradyarrhythmia, it has long been known that certain pharmaceuticals cause arrhythmia. Furthermore, it has recently been reported that sphingosine-1-phosphate (S1P) receptor modulators such as fingolimod and siponimod, which are used as therapeutic agents for multiple sclerosis, etc., induce bradyarrhythmia (e.g., Non-Patent Documents 1 and 2).

The pathogenic mechanism of such drug-induced bradyarrhythmia has not yet been fully elucidated. Also, the pathogenic mechanism of bradyarrhythmia by S1P receptor modulators has not yet been fully elucidated. For example, it has been reported that the pathogenic mechanism of bradyarrhythmia induced by fingolimod (i.e., an S1P receptor modulator) may be caused by activation of a cardiac acetylcholine-activated potassium channel (KACh channel) with which S1P1 or S1P3 (i.e., an S1P receptor subtype) is involved (e.g., Non-Patent Document 3) or direct channel inhibitory action by fingolimod (e.g., Non-Patent Documents 4 and 5). However, the pathogenic mechanism (including the probability described above) has not been fully elucidated, and no drug is known to be effective in treating such drug-induced bradyarrhythmia.

In addition, β-blockers, calcium antagonists, digitalis formulations, etc. are known to induce bradycardia, and they may cause therapeutic problems. It is desirable to use a β-blocker for the treatment of, for example, heart failure at a low dose at first, and to increase the dose step by step while monitoring drug tolerance (e.g., heart rate). The effects of β-blockers have been shown to be dose-responsive. In order to investigate the optimal dose of carvedilol (i.e., β-blocker) in Japanese patients with mild to moderate chronic heart failure, two doses [5 mg/day and 20 mg/day (twice a day)] were comparatively evaluated for efficacy and safety in the MUCHA trial. According to the test results, it was considered desirable to increase the dose to a high level, since an improvement in left ventricular ejection fraction was observed in a dose dependent manner. When a patient exhibits symptoms of cerebral ischemia associated with bradycardia or symptoms of heart failure associated with bradycardia at the start of administration of a β-blocker and during increase of the dose thereof, the dose of the drug must be reduced or the drug administration must be stopped. However, when a drug such as a β-blocker is effective in treating heart failure and continuation or dose increase is considered to be essential, implanting a pacemaker may be taken into consideration (e.g., Non-Patent Documents 6 and 7).

In fact, it has been reported that, in cardiac resynchronization therapy (CRT) (i.e., a pacemaker therapy for heart failure), a β-blocker can be orally administered at a high dose for a long period of time after CRT implantation, and the long-term administration is associated with prolongation of survival (e.g., Non-Patent Document 8). In this case, it is even more desirable if the drug can treat bradycardia.

Therefore, demand has arisen for development of new therapeutic agents or preventive agents for drug-induced bradycardia and bradyarrhythmia.

### Prior Art Documents

### Non-Patent Documents

Non-Patent Document 1: Journal of the American Society of Nephrology, 2002; 13: 1073-1083.
Non-Patent Document 2: British Journal of Clinical Pharmacology, 2013; 75: 831-841.
Non-Patent Document 3: American Journal of Transplantation, 2005; 5: 529-36.
Non-Patent Document 4: Toxicology and Applied Pharmacology, 2014; 15: 281: 39-47.
Non-Patent Document 5: Fundamental & Clinical Pharmacology, 2017; 31: 392-402.
Non-Patent Document 6: Japanese Circulation Society/Japanese Heart Failure Society Guideline, Guidelines for Diagnosis and Treatment of Acute and Chronic Heart Failure (2017 Revised Edition), 58-59.
Non-Patent Document 7: American Heart Journal, 2004; 147: 324-330.
Non-Patent Document 8: Optimization of Heart Failure Medication After Cardiac Resynchronization Therapy and the Impact on Long-Term Survival, European Heart Journal Cardiovascular Pharmacotherapy. 2015; 1(3): 182-188.

### SUMMARY OF THE INVENTION

### Problems to be Solved by the Invention

An object of the present invention is to provide a novel therapeutic agent for drug-induced bradycardia and bradyarrhythmia.

### Means for Solving the Problems

The aforementioned KACh channel is a cardiac acetylcholine-activated potassium channel. When acetylcholine is released from the vagus nerve terminal, the KACh channel opens through activation of M2 receptors, which are muscarinergic acetylcholine receptors, and potassium ions flow out of the cell, to thereby control the heart rate at a constant level. In particular, the channel is responsible for the physiological action of reducing the heart rate during parasympathetic activation.

(3R,4S)-7-Hydroxymethyl-2,2,9-trimethyl-4-(phenethylamino)-3,4-dihydro-2H-pyrano[2,3-g]quinoline-3-ol (hereinafter may be referred to as "compound (I)" or "the compound of the present invention") is a compound that has an atrial-selective refractory period prolonging action and has been reported to be effective for arrhythmia (e.g., atrial fibrillation) (see, for example, WO 2005/090357). The present inventors have found that compound (I) has a KACh channel inhibitory action.

As described above, the compound of the present invention is known to have an anti-atrial fibrillation action by a KACh channel inhibitory action. Atrial fibrillation, which often causes high tachycardia during sympathetic nerve activation, and drug-induced bradyarrhythmia can be said to be almost opposite pathological conditions.

However, the present inventors have conducted extensive studies, and as a result have found that, surprisingly, the compound of the present invention has high preventive effect and termination effect on drug-induced bradyarrhythmia.

Therefore, the present invention is as follows.
(1) A therapeutic agent for drug-induced bradycardia and bradyarrhythmia comprising, as an active ingredient, the following compound (I): 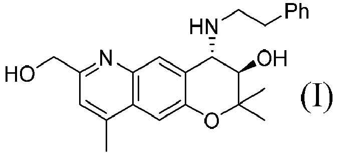 (wherein Ph is a phenyl group) or a pharmacologically acceptable salt of the compound.
(2) The therapeutic agent according to (1), wherein the drug-induced bradyarrhythmia is bradyarrhythmia induced by an S1P receptor modulator.
(3) The therapeutic agent according to (1), wherein the drug-induced bradyarrhythmia is bradyarrhythmia induced by an S1P1 modulator.
(4) The therapeutic agent according to (1), wherein the drug-induced bradyarrhythmia is bradyarrhythmia induced by fingolimod.
(5) The therapeutic agent according to (1), wherein the drug-induced bradyarrhythmia is bradyarrhythmia induced by siponimod.
(6) The therapeutic agent according to (1), wherein the drug-induced bradyarrhythmia is bradyarrhythmia induced by a β-blocker.
(7) The therapeutic agent according to (1), wherein the drug-induced bradyarrhythmia is bradyarrhythmia induced by propranolol.
(8) The therapeutic agent according to (1), wherein the drug-induced bradyarrhythmia is any of the following bradycardias: (a) sinus bradycardia, (b) sinus arrest, (c) sinoatrial block, (d) atrioventricular block, (e) sinus node dysfunction, (f) bradycardic heart failure, and (g) bradycardic atrial fibrillation.
(9) The therapeutic agent according to (1), wherein the drug-induced bradyarrhythmia is sinus bradycardia or atrioventricular block.
(10) The therapeutic agent according to (1), wherein the drug-induced bradyarrhythmia is sinus bradycardia.
(11) The therapeutic agent according to (1), wherein the drug-induced bradyarrhythmia is atrioventricular block.
(12) The therapeutic agent according to (1), wherein the therapeutic agent is for the purpose of prevention.
(13) The therapeutic agent according to (1), wherein the therapeutic agent is for the purpose of termination.
(14) A therapeutic agent for drug-induced bradycardia and bradyarrhythmia comprising, as an active ingredient, a compound having a KACh channel inhibitory action or a pharmacologically acceptable salt of the compound.
(15) The therapeutic agent according to any one of (1) to (14), wherein the therapeutic agent for drug-induced bradycardia and bradyarrhythmia is a therapeutic agent for drug-induced bradyarrhythmia.
(16) The therapeutic agent according to any one of (1) to (14), wherein the therapeutic agent for drug-induced bradycardia and bradyarrhythmia is a therapeutic agent for bradyarrhythmia.

### Effects of the Invention

The present invention provides a therapeutic agent for drug-induced bradycardia and bradyarrhythmia.

### BRIEF DESCRIPTION OF THE DRAWINGS

[FIG. 1] FIG. 1 is graphs showing the effect of compound (I) on fingolimod-induced bradyarrhythmia in anesthetized guinea pigs.
[FIG. 2] FIG. 2 is representative electrocardiographic waveforms obtained upon evaluation of the preventive effect of compound (I) on fingolimod-induced bradyarrhythmia in anesthetized guinea pigs.
[FIG. 3] FIG. 3 is graphs showing the effect of compound (I) on siponimod-induced bradyarrhythmia in anesthetized guinea pigs.
[FIG. 4] FIG. 4 is representative electrocardiographic waveforms obtained upon evaluation of the preventive effect of compound (I) on siponimod-induced bradyarrhythmia in anesthetized guinea pigs.
[FIG. 5] FIG. 5 is representative electrocardiographic waveforms obtained when compound (I) exhibited the effect of terminating fingolimod-induced bradyarrhythmia in anesthetized guinea pigs.
[FIG. 6] FIG. 6 is a graph showing the effect of compound (I) on siponimod-induced decrease in beating rate in iPS cell-derived atrial myocytes.
[FIG. 7] FIG. 7 is graphs showing the effect of compound (I) on fingolimod-induced decrease in peripheral blood lymphocytes in guinea pigs.
[FIG. 8] FIG. 8 shows heart rates before and after administration of compound (I) or saline to awake rats upon evaluation of the therapeutic effect of compound (I) or saline on propranolol-induced bradyarrhythmia by telemetry (error bars correspond to standard errors).
[FIG. 9] FIG. 9 is graphs showing the effect of compound (I) on propranolol-induced bradycardia in awake beagle dogs.
[FIG. 10] FIG. 10 is a representative electrocardiographic waveform corresponding to verapamil-induced bradyarrhythmia (2:1 atrioventricular block) in an awake beagle dog and a representative electrocardiographic waveform obtained upon conversion into Wenckebach atrioventricular block and subsequent recovery to 1:1 atrioventricular conduction by administration of compound (I).

### MODES FOR CARRYING OUT THE INVENTION

As described above, the heart rate of humans at rest is generally about 50 beats/minute to 100 beats/minute, and bradycardia is a heart rate lower than that. No particular limitation is imposed on the term "bradyarrhythmia" as used herein, which is a concept well known to those skilled in the art. In one embodiment, bradyarrhythmia is a disease that is clinically diagnosed as bradycardia. More specifically, bradycardia to be treated with the compound of the present invention is a pathological condition in which symptoms of heart failure are improved by increasing heart rate and cardiac output with the compound of the present invention.

As described above, drug-induced bradyarrhythmia refers to a condition in which the aforementioned bradyarrhythmia is induced by a drug administered for the purpose of treating a certain disease.

Examples of drugs that are known to induce bradycardia include a β-blocker, a non-dihydropyridine calcium antagonist, a digitalis formulation, and an S1P receptor modulator.

The S1P receptor modulator refers to a modulator of sphingosine-1-phosphate (S1P) receptor. The S1P receptor modulator is typically an antagonist of S1P receptor. The S1P receptor modulator is typically a functional antagonist of S1P receptor. No particular limitation is imposed on the S1P receptor modulator in the present invention, so long as it is a drug that affects an S1P receptor. In the most typical embodiment, the S1P receptor modulator in the present invention is a drug that affects an S1P receptor and induces bradycardia. In one embodiment, the S1P receptor modulator in the present invention is fingolimod, siponimod, cenerimod, ponesimod, or ozanimod.

The β-blocker is a drug that exhibits a blocking action on only a β-receptor among sympathetic adrenergic receptors. The β-blocker is also referred to as a beta-blocker. No particular limitation is imposed on the β-blocker in the present invention, so long as it is a drug that has a blocking action on a sympathetic β-adrenergic receptor. In the most typical embodiment, the β-blocker in the present invention is a drug that has a β-blocking action and induces bradycardia. In one embodiment, the β-blocker in the present invention is carvedilol, bisoprolol, metoprolol, atenolol, propranolol, landiolol, arotinolol, pindolol, labetalol, alprenolol, nadolol, acebutolol, carteolol, or bevantolol. The β-blocker in the present invention is more preferably carvedilol, bisoprolol, metoprolol, atenolol, propranolol, or arotinolol.

The calcium antagonist is a drug that acts on an L-type Ca channel, which is one of membrane voltage-dependent Ca channels, to thereby reduce the entry of Ca ions into cells. Based on the difference in chemical structure, calcium antagonists are classified into dihydropyridine compounds (nifedipine, nicardipine, amlodipine, felodipine, nitrendipine, nilvadipine, manidipine, barnidipine, cilnidipine, benidipine, azelnidipine, aranidipine, and efonidipine), benzothiazepine compounds (diltiazem), and phenylalkylamine compounds (verapamil). A dihydropyridine Ca antagonist mainly prevents the entry of Ca ions into vascular smooth muscles and dilates blood vessels, to thereby exert a hypotensive effect. Thus, a dihydropyridine Ca antagonist is applied to hypertension. A dihydropyridine Ca antagonist lowers blood pressure mainly by dilating peripheral blood vessels, whereas a non-dihydropyridine Ca antagonist mainly dilates the coronary artery and acts on the myocardium. A non-dihydropyridine Ca antagonist mainly antagonistically inhibits the entry of Ca ions into Ca channels of the coronary artery, to thereby dilate the coronary artery and ameliorate angina pectoris. The entry of Ca ions is responsible for the heartbeat, and a non-dihydropyridine Ca antagonist inhibits the entry of Ca ions into myocardial cells to thereby ameliorate tachyarrhythmia. No particular limitation is imposed on the calcium antagonist in the present invention, so long as it is a drug that acts on voltage-dependent Ca channels in blood vessels or myocardial cells to thereby suppress the entry of Ca ions into cells. In the most typical embodiment, the calcium antagonist in the present invention is a drug that has a vasodilating effect and exhibits an antitachycardia action for suppressing cardiac contractility and excitation conduction of the sinoatrial node/atrioventricular node to thereby reduce heart rate. In one embodiment, the calcium antagonists in the present invention are nifedipine, nicardipine, amlodipine, felodipine, nitrendipine, nilvadipine, manidipine, barnidipine, cilnidipine, benidipine, azelnidipine, aranidipine, efonidipine, diltiazem, and verapamil.

For example, regarding S1P receptor modulators, fingolimod is required, in its package insert, to satisfy the condition that "a decrease in heart rate is observed for several days after the start of administration of the drug" and that "the heart rate may lower significantly particularly in the early stage of administration, and thus administration should be started under supervision that allows appropriate treatment (e.g., cooperation with a doctor who specializes in the cardiovascular system)."

Among S1P receptor modulators, fingolimod, siponimod, cenerimod, ponesimod and ozanimod have each been reported to affect the heart rate. All of these drugs are known to act on S1P1.

Therefore, the drug-induced bradyarrhythmia in the present invention is understood as the following diseases.
(1) Drug-induced bradyarrhythmia
(2) S1P receptor modulator-induced bradyarrhythmia
(3) S1P1 modulator-induced bradyarrhythmia

As described below, the present inventors have found that the compound of the present invention is effective for bradyarrhythmia induced by fingolimod or siponimod.

Therefore, the drug-induced bradyarrhythmia in the present invention is understood as the following diseases.
(4) Fingolimod-induced bradyarrhythmia
(5) Siponimod-induced bradyarrhythmia

Regarding β-blockers, the package insert of carvedilol describes "severe bradycardia" and "complete atrioventricular block" as "serious side effects."

Similarly, the package insert of bisoprolol describes "severe bradycardia," "complete atrioventricular block," and "sick sinus syndrome" as "serious side effects."

In addition, arrhythmic side effects described in the package inserts of other β-blockers are as follows: the package insert of metoprolol describes atrioventricular block, bradycardia, and sinus node dysfunction; the package insert of atenolol describes bradycardia, atrioventricular block, and sinoatrial block; the package insert of propranolol describes bradycardia and atrioventricular block; and the package insert of landiolol describes complete atrioventricular block, sinus arrest, and severe bradycardia.

As described below, the present inventors have also found that the compound of the present invention is effective for bradyarrhythmia induced by propranolol. A β-blocker is a drug that exhibits a blocking action on β-receptors among sympathetic adrenergic receptors, and its bradycardia action is also based on the β-blocking action. Therefore, the compound of the present invention is equally effective for bradycardia induced by all β-blockers.

A β-blocker is a drug with a wide range of applications, including hypertension, heart failure, angina pectoris, and heart rate control in atrial fibrillation.

Hitherto, no therapy has been established for reversing β-blocker-induced bradycardia with other drugs.

As described above, it has been reported that to a patient having heart failure with β-blocker-induced bradyarrhythmia when a β-blocker is continuously administered while improving bradycardia by cardiac resynchronization therapy, etc., prolongation of the patient's survival can be expected.

Thus, the present inventors have conceived that if a drug capable of improving bradycardia without affecting actions (other than heart rate lowering action) of a β-blocker (e.g., cardiac contractility suppressing action), i.e., "therapeutic agent for drug-induced bradyarrhythmia" can be developed, chronic heart failure can be treated by oral administration of the therapeutic agent in combination with a bradycardia-inducing drug (e.g., β-blocker), or by oral administration of a mixture of the therapeutic agent for drug-induced bradyarrhythmia and a bradycardia-inducing drug (e.g., β-blocker).

As for angina pectoris, the purpose of administration of a β-blocker is to reduce myocardial oxygen consumption by suppressing contraction, and the main purpose is not to reduce heart rate. Thus, in the case of angina pectoris, it is quite possible and probably appropriate to use the compound of the present invention for lowering contractility and blood pressure while maintaining the heart rate.

With regard to the improvement of bradycardia induced by the use of a β-blocker for heart rate control in atrial fibrillation, the present invention is expected to improve bradycardia particularly at night when the parasympathetic nerve is dominant. Thus, the present invention is appropriate for the treatment of atrial fibrillation.

Therefore, the bradycardia treatment (correction) with the compound of the present invention is considered appropriate for bradycardia induced by the use of a β-blocker for heart failure, hypertension, angina pectoris, and heart rate control in atrial fibrillation.

Thus, in one embodiment, the drug-induced bradyarrhythmia in the present invention is understood as the following diseases.
(6) β-Blocker-induced bradyarrhythmia
(7) Propranolol-induced bradyarrhythmia

A calcium antagonist is a drug that is widely used as an antihypertensive, an antiarrhythmic agent, and an antianginal agent. The actions of a calcium antagonist are classified into dilation of blood vessels (including coronary artery), suppression of cardiac contractility, and inhibition of the impulse conducting system. Calcium antagonists are roughly classified into dihydropyridine compounds, benzothiazepine compounds, and phenylalkylamine compounds. There are many types of dihydropyridine calcium antagonists, which are mainly used for hypertension treatment. Examples of dihydropyridine calcium antagonists include nifedipine, nicardipine, amlodipine, and cilnidipine. Diltiazem is a representative benzothiazepine calcium antagonist, and verapamil is a representative phenylalkylamine calcium antagonist.

Since a phenylalkylamine calcium antagonist such as verapamil has an inhibitory action (negative chronotropic effect) on the sinus node or the atrioventricular node, the phenylalkylamine calcium antagonist is used as an antiarrhythmic agent for tachyarrhythmia such as supraventricular tachycardia. However, the phenylalkylamine calcium antagonist has been reported to cause serious side effects such as sinus arrest, atrioventricular block, and bradycardia. Also, the phenylalkylamine calcium antagonist is contraindicated in a patient with congestive heart failure, because of its strong negative inotropic effect (cardiac contractility-suppressing action).

Thus, the present inventors have conceived that if a drug capable of improving bradycardia, i.e., "therapeutic agent for drug-induced bradyarrhythmia" can be developed for bradyarrhythmia (i.e., side effects) induced by verapamil used as a therapeutic agent for tachyarrhythmia such as supraventricular tachycardia and bradyarrhythmia associated with bradycardia tachycardia syndrome, such a bradyarrhythmia condition can be treated by oral administration of the therapeutic agent for drug-induced bradyarrhythmia in combination with verapamil, or by oral administration of a mixture of the therapeutic agent for drug-induced bradyarrhythmia and verapamil.

In general, calcium antagonists (including but not limiting to verapamil) have a negative chronotropic effect, a vasodilating effect (hypotensive action), and a negative inotropic effect. It is thought to be quite possible and appropriate to use the compound of the present invention as "therapeutic agent for drug-induced bradyarrhythmia" for the prevention and treatment of excessive bradycardia or atrioventricular block due to the negative chronotropic effect. Therefore, the bradycardia treatment (correction) with the compound of the present invention is considered appropriate for bradycardia induced by the use of a calcium antagonist for supraventricular tachyarrhythmia such as atrial fibrillation, hypertension, and angina pectoris.

Thus, in one embodiment, the drug-induced bradyarrhythmia in the present invention is understood as the following diseases.
(8) Calcium antagonist-induced bradyarrhythmia
(9) Verapamil-induced bradyarrhythmia

The aforementioned bradycardia-inducing drugs in the present invention, including β-blocker, S1P receptor modulator, digitalis formulation, and calcium antagonist include existing drugs, existing drugs that will be found to have such actions in the future, and new drugs that will be found to have such actions in the future.

No limitation is imposed on the drug-induced bradycardia and bradyarrhythmia to be treated with the therapeutic agent of the present invention, so long as the therapeutic agent is effective therefor.

As described above, the present inventors have found that the compound of the present invention ameliorates bradyarrhythmia induced by each of a β-blocker, a calcium antagonist, and an S1P receptor modulator, which have completely different bradyarrhythmia-inducing mechanisms. Thus, the compound of the present invention is effective for drug-induced bradyarrhythmias induced by the mechanisms of all drugs, including, but not limited to, a β-blocker, a calcium antagonist, and an S1P receptor modulator.

The present inventors have found that the compound of the present invention is effective for sinus arrest and atrioventricular block in a genetically modified animal model.

As described above, bradycardia induced by a β-blocker and a calcium antagonist includes bradycardia, severe bradycardia, complete atrioventricular block, atrioventricular block, sick sinus syndrome, sinus node dysfunction, sinus arrest, and severe bradycardia, and bradycardia induced by an S1P receptor modulator includes a reduction in heart rate over several days.

The present inventors have demonstrated the efficacy of the compound of the present invention for a β-blocker, a calcium antagonist, and an S1P receptor modulator that induce these various types of bradycardia.

Thus, symptoms targeted by the therapeutic agent of the present invention may include the following.
(8) Any of the following types of bradycardia:
   (a) sinus bradycardia, (b) sinus arrest, (c) sinoatrial block, (d) atrioventricular block, (e) sinus node dysfunction, (f) bradycardic heart failure, and (g) bradycardic atrial fibrillation.

As described below, the present inventors have also found that the compound of the present invention is particularly effective for drug-induced atrioventricular block or sinus bradycardia.

Thus, the drug-induced bradyarrhythmia for which the compound of the present invention is particularly effective is understood as the following diseases.
(9) Either sinus bradycardia or atrioventricular block
(10) Sinus bradycardia
(11) Atrioventricular block

In the present invention, sinus bradycardia is a pathological condition in which the pulsation rhythm is normal and regular, but the heart rate slows down among arrhythmia symptoms. Generally, sinus bradycardia is understood as a pathological condition in which the heart rate decreases to 50 beats or less per minute. Sinus bradycardia is understood as a pathological condition of Group I among sick sinus syndromes according to Rubenstein classification.

In the present invention, sinus arrest is a pathological condition in which no electronic signal is generated from the sinus node, and atrial activation does not occur.

In the present invention, sinoatrial block is a pathological condition in which atrial activation does not occur due to impaired excitation conduction between the sinus node and the atrium.

In the present invention, atrioventricular block is a pathological condition in which ventricular activation does not occur due to impaired excitation conduction between the atrium and the ventricle. Atrioventricular block includes the following disease type classifications.
(1) First-degree atrioventricular block
   The conduction time between the atrium and the ventricle is prolonged (excitation conduction is maintained).
(2) Second-degree atrioventricular block: the conduction between the atrium and the ventricle is suddenly interrupted. It includes the following four types.
   (2-1) Wenckebach type: ventricular activation is lost after the conduction between the atrium and the ventricle is gradually prolonged.
   (2-2) Mobitz type II: ventricular activation is suddenly lost without prolongation of the conduction between the atrium and the ventricle.
   (2-3) 2:1 Atrioventricular block: block with an atrioventricular conduction ratio of 2: 1.
   (2-4) Advanced atrioventricular block: block with an atrioventricular conduction ratio of 3 : 1 or more.
(3) Third-degree atrioventricular block: the conduction between the atrium and the ventricle is completely interrupted.

In the present invention, sinus node dysfunction refers to impaired excitation conduction from the sinus node to the atrium, and refers to several pathological conditions that result in atrial activation at physiologically inappropriate heart rates. Sick sinus syndromes are as follows: inappropriate sinus bradycardia, alternating bradycardia and atrial tachyarrhythmia (bradycardia-tachycardia syndrome), sinus arrest or sinus pause, and sinoatrial block.

In the present invention, bradycardic heart failure refers to a clinical syndrome in which dyspnea, fatigue, and edema occur as a result of failure to maintain the circulating blood volume required for physical activities due to an excessive decrease in heart rate, and the failure of compensatory mechanism of the heart pump function, whereby exercise tolerance is reduced. The "bradycardia" as used herein does not necessarily have to be a heart rate of 50 beats or less, and pathological conditions in which heart failure symptoms may be improved by increasing the heart rate (including bradycardic heart failure) are to be treated with the therapeutic agent of the present invention.

In the present invention, bradycardic atrial fibrillation refers to a pathological condition in which the function of the atrioventricular node that conducts electrical excitation from the atrium to the ventricle is impaired for some reason in a patient with atrial fibrillation, which is complicated with atrioventricular block due to decreased atrioventricular conduction, resulting in ventricular bradycardia.

As described below, the present inventors have also found that the compound of the present invention is particularly effective for the prevention or termination of drug-induced bradyarrhythmia. Thus, the therapeutic agent for drug-induced bradyarrhythmia in the present invention is understood as the following therapeutic agents.
(12) Therapeutic agent for the purpose of prevention.
(13) Therapeutic agent for the purpose of termination.

Prevention is a concept well known to those skilled in the art. In one embodiment, prevention is "to prevent bad things from happening previously" (Kojien). Therefore, the "therapeutic agent for the purpose of prevention of drug-induced bradyarrhythmia" of the present invention is a "therapeutic agent for the purpose of preventing drug-induced bradyarrhythmia previously so that it does not occur" in one embodiment.

No particular limitation is imposed on the definition of the term "previously" as used herein, so long as the prevention is performed at a time before drug-induced bradyarrhythmia occurs. Thus, the administration of the compound of the present invention for the purpose of prevention may be performed before or after administration of a compound that induces arrhythmia (hereinafter the compound will be referred to as "inducer"). Alternatively, the administration of the compound of the present invention may be performed a plurality of times before and after administration of the inducer.

The first administration of the compound of the present invention for the purpose of prevention is performed before administration of the inducer in one embodiment, up to one day before administration of the inducer in one embodiment, or up to 12 hours before administration of the inducer in one embodiment.

The administration for the purpose of prevention is determined in a clinical setting in consideration of the pharmacokinetics of the inducer and the compound of the present invention. Thus, when the administration is understood to be "for the purpose of prevention" by those skilled in the art in a clinical setting, all cases fall within the category of prevention. The category of prevention described herein also includes the case where it is difficult to administer a drug that causes bradyarrhythmia (e.g., an S1P receptor modulator or a β-blocker) at a required effective dose for an indication (e.g., an S1P receptor modulator for multiple sclerosis, or a β-blocker for chronic heart failure) due to the onset of bradyarrhythmia, and the case where the administration of such a drug can be started or the dose of the drug can be increased when the drug is used in combination with the compound of the present invention.

Termination is a concept well known to those skilled in the art. In one embodiment, termination is "to temporarily stop a movement" (Kojien). Therefore, the "therapeutic agent for the purpose of termination of drug-induced bradyarrhythmia" of the present invention is a "therapeutic agent for the purpose of stopping drug-induced bradyarrhythmia" in one embodiment. When the administration is understood to be "for the purpose of termination" by those skilled in the art in a clinical setting, all cases fall within the category of termination.

As described above, the compound of the present invention is a compound that has an atrial-selective refractory period prolonging action and has been reported to be effective for arrhythmia (e.g., atrial fibrillation). When the compound of the present invention is used in combination with an inducer, rather than when the compound of the present invention is expected to be effective as a single drug, the administration of the compound is presumed for the purpose of treatment or prevention or termination of drug-induced bradyarrhythmia.

As described above, the compound of the present invention (compound (I)) has a KACh channel inhibitory action, and may exhibit the effects of the present invention because of this action. Thus, any compound having a KACh channel inhibitory action (not limited to compound (I)) is highly likely to exhibit the effects of the present invention. Therefore, in one embodiment, the therapeutic agent of the present invention is as follows. (14) Therapeutic agent for drug-induced bradycardia and bradyarrhythmia containing, as an active ingredient, a compound having a KACh channel inhibitory action or a pharmacologically acceptable salt of the compound.

In one embodiment, the present invention provides a therapeutic agent for drug-induced bradycardia and bradyarrhythmia. As described herein, the compound of the present invention is effective for both drug-induced bradyarrhythmia and non-drug-induced bradyarrhythmia. Therefore, the therapeutic agent for drug-induced bradycardia and bradyarrhythmia of the present invention is understood as therapeutic agents described below. As described in Examples, the compound of the present invention has the effect of increasing heart rate and cardiac output even when the initial heart rate is 50 beats or more. Thus, the term "bradyarrhythmia" as used herein includes pathological conditions in which the present compound may increase the heart rate and improve symptoms of heart failure, whether or not a heart rate is 50 beats per minute or less.
(15) Therapeutic agent for drug-induced bradyarrhythmia.
(16) Therapeutic agent for bradyarrhythmia.

In one embodiment, preferably, the therapeutic agent for drug-induced bradycardia does not affect the drug efficacy of the drug in view of the aforementioned clinical setting. For example, a therapeutic agent for S1P receptor modulator-induced bradycardia preferably does not inhibit the peripheral blood lymphocyte decreasing action of the S1P receptor modulator. However, there are many unclear points about the mutual influence between drug efficacies of a plurality of drugs, and it is difficult to predict the presence or absence of the interaction between the drug efficacies. Surprisingly, the present inventors have also found that compound (I) does not inhibit the peripheral blood lymphocyte decreasing action of a S1P receptor modulator.

As described above, compound (I) contained in the therapeutic agent of the present invention is (3R,4S)-7-hydroxymethyl-2,2,9-trimethyl-4-(phenethylamino)-3,4-dihydro-2H-pyrano[2,3-g]quinoline-3-ol.

In one embodiment, compound (I) may be a racemic mixture or diastereomeric mixture containing a (3R,4S) form.

In another embodiment, the compound contained in the therapeutic agent of the present invention may be an analogue of compound (I). Examples of the analogue include the compound described in WO 2005/090357.

Compound (I) contained in the therapeutic agent of the present invention may be in the form of a pharmacologically acceptable salt.

Examples of the pharmacologically acceptable salt include hydrochloride, hydrobromide, sulfate, methanesulfonate, acetate, benzoate, tartrate, phosphate, lactate, maleate, fumarate, malate, gluconate, and salicylate.

Preferable salts of compound (I) are hydrochloride, maleate, and methanesulfonate.

The compound contained in the therapeutic agent of the present invention may be in the form of a crystal. The crystal may have any crystal form so long as it does not impair the object of the present invention. Examples of preferable crystal forms of compound (I) include crystal forms described in WO 2010/126138. In one embodiment, the crystal is a crystal having a characteristic peak at a diffraction angle 2θ = 5.6, 8.2, 12.0, 14.7, 16.6, 16.9, 17.9, 18.4, 22.5, 24.5, 27.6 (± 0.2°) as a powder X-ray diffraction pattern when Cu·Kα is used as an X-ray source. In another embodiment, the crystal is a crystal having any 3, 5, 7 or 9 peaks among the aforementioned 11 peaks. Identification of the crystal form by powder X-ray diffractometry is performed based on common technical knowledge of those skilled in the art, for example, description of Japanese Pharmacopeia. When X-rays other than Cu·Kα rays are used, diffraction angle patterns are compared according to conversion of the 2θ value based on the Bragg's equation. The measurement error of the 2θ value is generally in a range of ± 0.2°, but even if there are a small number of peaks having an error of 0.2° or more, it does not hinder rational identification for those skilled in the art.

Compound (I) contained in the therapeutic agent of the present invention can be produced by any known method. Examples of the known method include methods described in WO 2005/090357 and WO 2010/126138. Compound (I) can be produced with reference to, for example, WO 2007/105658, WO 2014/050613, WO 2014/051077, or WO 2015/012271.

The present invention provides a pharmaceutical composition or veterinary pharmaceutical composition containing an effective amount of the compound of the present invention for the aforementioned treatment.

Examples of dosage forms of the compound according to the present invention include parenteral administration with injection agents (subcutaneous, intravenous, intramuscular, intraperitoneal injection), ointments, suppositories, aerosol agents, and the like or oral administration with tablets, capsules, granules, pills, syrups, liquids, emulsions, suspensions, and the like.

The pharmaceutical or veterinary composition containing the compound according to the present invention contains the compound according to the present invention in an amount of about 0.01% to 99.5%, preferably about 0.1% to 30%, relative to the weight of the entire composition.

In addition to the compound according to the present invention or a composition containing the compound, other pharmaceutical or veterinary active compounds can be incorporated.

Such a composition can contain a plurality of compounds according to the present invention.

The clinical dose of the compound of the present invention varies depending on the age, body weight, patient sensitivity, the degree of symptoms, etc., but the effective dose is generally 0.003 g to 1.5 g, preferably about 0.01 g to 0.6 g per day for adults. However, if necessary, the compound may be used in an amount falling outside the aforementioned range.

The compound of the present invention is formulated for administration by conventional pharmaceutical means.

Specifically, tablets, capsules, granules, and pills for oral administration are prepared using excipients, for example, white sugar, lactose, glucose, starch, and mannitol; binders, for example, hydroxypropyl cellulose, syrup, gum arabic, gelatin, sorbitol, tragacanth, methyl cellulose, and polyvinylpyrrolidone; disintegrants, for example, starch, carboxymethyl cellulose or its calcium salt, microcrystalline cellulose, and polyethylene glycol; smoothing agents, for example, talc, magnesium or calcium stearate, and silica; and lubricants, for example, sodium laurate, and glycerol.

Injection agents, liquids, emulsions, suspensions, syrups and aerosol agents are prepared using active ingredient solvents, for example, water, ethyl alcohol, isopropyl alcohol, propylene glycol, 1,3-butylene glycol, and polyethylene glycol; surfactants, for example, sorbitan fatty acid ester, polyoxyethylene sorbitan fatty acid ester, polyoxyethylene fatty acid ester, polyoxyethylene ether of hydrogenated castor oil, and lecithin; suspensions, for example, carboxymethyl sodium salts, cellulose derivatives such as methyl cellulose, and natural rubbers such as tragacanth and gum arabic; preservatives, for example, paraoxybenzoic acid ester, benzalkonium chloride, and sorbate, and the like.

For the ointment (i.e., a percutaneous absorption type formulation), for example, white petrolatum, liquid paraffin, higher alcohols, macrogol ointments, hydrophilic ointments, or aqueous gel bases are used.

Suppositories are prepared using, for example, cacao fat, polyethylene glycol, lanolin, fatty acid triglyceride, coconut oil, or polysorbate.

### Examples

The present invention will next be described in more detail by way of Examples, but the present invention should not be construed as being limited to the following Examples.

### Evaluation Example 1

### Preventive Effect of Compound (I) on Fingolimod-Induced Bradyarrhythmia in Isoflurane-Anesthetized Guinea Pig Model

A solvent or compound (I) (free form) was administered by oral gavage at a dose of 1, 3, or 10 mg/kg (volume: 5 mL/kg) to Hartley guinea pigs (7 to 10 weeks old, male, available from Japan SLC, Inc.). After completion of the administration, each animal was subjected to anesthetic induction by inhalation of isoflurane (available from Pfizer Japan Inc.), and the animal was held on an operating table having a heater function. The trachea was incised, and a tracheal cannula (KN-375, available from Natsume Seisakusho Co., Ltd.) was inserted. The tracheal cannula was connected to a ventilator (SN-480-7, available from Shinano Seisakusho Co., Ltd.), and artificial respiration was controlled by supplying 100% oxygen at a volume of 10 mL and a ventilation rate of 60 times/minute. The solvent used was 0.5 w/v% methyl cellulose 400 solution (available from FUJIFILM Wako Pure Chemical Corporation). Isoflurane (available from Pfizer Japan Inc.) anesthesia (about 1 to 4%, carrier gas: 100% oxygen) was used for maintenance of anesthesia. A catheter (BD intramedic polyethylene tubing PE50, available from Becton, Dickinson and Company) charged with 50 units/mL heparin (available from AY PHARMACEUTICALS CO., LTD.) was inserted into each of the carotid artery and the jugular vein and placed in the blood vessel. Systemic blood pressure was measured with a high-sensitivity DC amplifier (AD-611J, available from NIHON KOHDEN CORPORATION) via the catheter placed in the carotid artery. Electrodes were attached to the body surface, and a body surface electrocardiogram was measured with a high-sensitivity amplifier (AB-611J, available from NIHON KOHDEN CORPORATION). Blood pressure and electrocardiogram data were acquired and analyzed with PowerLab/Labchart system (available from ADInstruments). Analysis items are systolic blood pressure, diastolic blood pressure, average blood pressure, heart rate, P-wave width, PR interval, QRS width, QT interval, and corrected QT interval. Corrected QT interval was calculated using the fridericia formula (QTc = QT interval/RR interval^{1/3}). After stabilization of each parameter, fingolimod hydrochloride (synthesized with reference to WO 2000/027798) was continuously administered intravenously at 0.1 mg/kg for 10 minutes via the catheter placed in the jugular vein, followed by observation of the presence or absence of the onset of bradyarrhythmia for 60 minutes. The solvent used was saline (available from Otsuka Pharmaceutical Factory, Inc.). Data were recorded and blood was collected 5, 10, 15, 20, 30, and 60 minutes after initiation of administration of fingolimod hydrochloride. The observation was terminated 60 minutes after initiation of the administration, and the animal was euthanized by exsanguination or intravenous administration of a large amount of pentobarbital or saturated potassium chloride solution under isoflurane anesthesia. Data were analyzed for atrial beating rate over time, ventricular beating rate over time, electrocardiographic parameters, the presence or absence of the onset of atrioventricular block, and plasma concentrations of compound (I), fingolimod, and fingolimod phosphorylated form. The plasma was collected by centrifuging the blood and cryopreserved at about -20°C. After thawing of the plasma, plasma drug concentrations were measured using LC/MS/MS after deproteinization with acetonitrile.

Atrioventricular block was defined as follows.

Second-degree atrioventricular block: atrial activation wave (P wave) is normal, which is followed by or not followed by ventricular activation wave (QRS wave).

Third-degree atrioventricular block: there is no electrical conduction between the atrium and the ventricle, and atrial and ventricular activation waves are generated independently, resulting in atrioventricular dissociation.

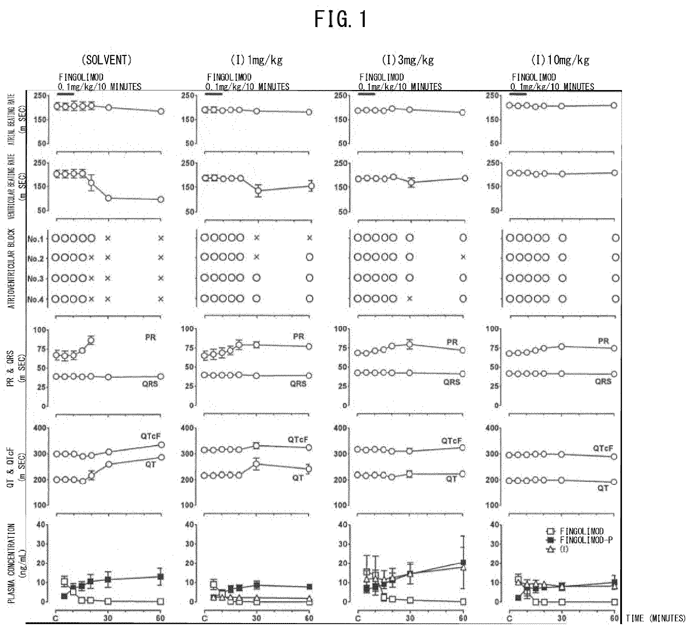

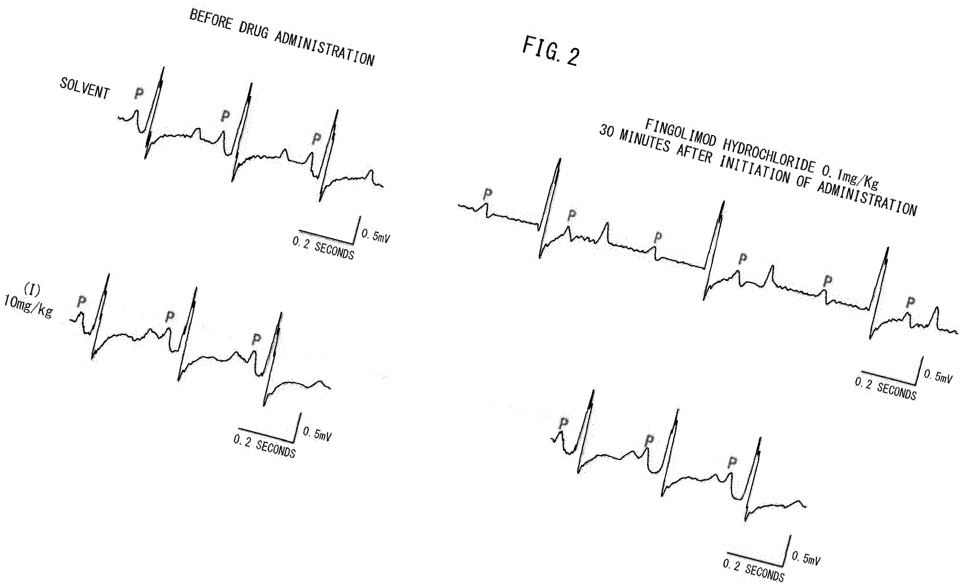

FIG. 1 shows changes over time in parameter measurements in each group, and FIG. 2 shows the resultant representative electrocardiographic waveforms. In FIG. 1, the measurements are shown as mean ± standard error. Abbreviations, etc. are as follows.
(I): compound (I)
Atrioventricular block: the presence or absence of atrioventricular block
(× denotes second- or third-degree atrioventricular block)
PR & QRS: atrioventricular conduction time and ventricular activation time
QT & QTcF: ventricular repolarization time and heart rate-corrected ventricular repolarization time
Plasma concentration: the plasma concentration of fingolimod, fingolimod phosphorylated form (-P), or compound (I)

FIG. 1 shows comparison of the effects of no administration of compound (I) and administration of compound (I) at doses of 1 mg/kg, 3 mg/kg, and 10 mg/kg. Regarding the ventricular beating rate, noticeable fingolimod-induced bradycardia was observed (e.g., about a half reduction in ventricular beating rate) in the case of no administration. In contrast, in the compound (I) administration group, bradycardia was improved in a dose-dependent manner. Specifically, bradycardia was almost restored in the 3 mg/kg administration group, and bradycardia was completely restored in the 10 mg/kg administration group.

Regarding the presence or absence of the onset of atrioventricular block, fingolimod-induced atrioventricular block was observed in all cases (four out of four cases) in the non-administration group. In contrast, atrioventricular block was not observed in three cases (75%) in the 1 mg/kg and 3 mg/kg administration groups of compound (I), and atrioventricular block was suppressed in four cases (100%) in the 10 mg/kg administration group. Similarly, fingolimod-induced prolongation of QT time was almost completely suppressed.

FIG. 2 shows typical electrocardiograms in the compound (I) non-administration group and the 10 mg/kg administration group. In the non-administration group, about a half reduction in heat rate was observed; i.e., noticeable bradycardia was observed. In contrast, in the 10 mg/kg administration group, such fingolimod-induced bradycardia was completely resolved.

As shown in FIG. 1 and FIG. 2, compound (I) prevented the onset of fingolimod-induced bradyarrhythmia (atrioventricular block) in a dose-dependent manner, and prevented a reduction in heart rate.

### Evaluation Example 2

### Preventive Effect of Compound (I) on Siponimod-Induced Bradyarrhythmia in Isoflurane-Anesthetized Guinea Pig Model

A solvent or compound (I) (free form) was administered by oral gavage at a dose of 0.3. 1, or 3 mg/kg (volume: 5 mL/kg) to Hartley guinea pigs (7 to 10 weeks old, male, available from Japan SLC, Inc.). After completion of the administration, each animal was subjected to anesthetic induction by inhalation of isoflurane (available from Pfizer Japan Inc.), and the animal was held on an operating table having a heater function. The trachea was incised, and a tracheal cannula (KN-375, available from Natsume Seisakusho Co., Ltd.) was inserted. The tracheal cannula was connected to a ventilator (SN-480-7, available from Shinano Seisakusho Co., Ltd.), and artificial respiration was controlled by supplying 100% oxygen at a volume of 10 mL and a ventilation rate of 60 times/minute. The solvent used was 0.5 w/v% methyl cellulose 400 solution (available from FUJIFILM Wako Pure Chemical Corporation). Isoflurane (available from Pfizer Japan Inc.) anesthesia (about 1 to 4%, carrier gas: 100% oxygen) was used for maintenance of anesthesia. A catheter (BD intramedic polyethylene tubing PE50, available from Becton, Dickinson and Company) charged with 50 units/mL heparin (available from AY PHARMACEUTICALS CO., LTD.) was inserted into each of the carotid artery and the jugular vein and placed in the blood vessel. Systemic blood pressure was measured with a high-sensitivity DC amplifier (AD-611J, available from NIHON KOHDEN CORPORATION) via the catheter placed in the carotid artery. Electrodes were attached to the body surface, and a body surface electrocardiogram was measured with a high-sensitivity amplifier (AB-611J, available from NIHON KOHDEN CORPORATION). Blood pressure and electrocardiogram data were acquired and analyzed with PowerLab/Labchart system (available from ADInstruments). Analysis items are systolic blood pressure, diastolic blood pressure, average blood pressure, heart rate, P-wave width, PR interval, QRS width, QT interval, and corrected QT interval. Corrected QT interval was calculated using the Fridericia formula (QTc = QT interval/RR interval^{1/3}). After stabilization of each parameter, siponimod (synthesized according to WO 2017/120124) was continuously administered intravenously at 0.01 mg/kg for 10 minutes via the catheter placed in the jugular vein, followed by observation of the presence or absence of the onset of bradyarrhythmia for 60 minutes. The solvent used was a solution prepared by mixing of Polyethylene Glycol 400 (available from KANTO CHEMICAL CO., INC.) and dimethyl sulfoxide (available from FUJIFILM Wako Pure Chemical Corporation) at 9 : 1 (volume ratio). Data were recorded and blood was collected 5, 10, 15, 20, 30, and 60 minutes after initiation of administration of siponimod. The observation was terminated 60 minutes after initiation of the administration, and the animal was euthanized by exsanguination or intravenous administration of a large amount of saturated potassium chloride solution under isoflurane anesthesia. Data were analyzed for atrial beating rate over time, ventricular beating rate over time, electrocardiographic parameters, the presence or absence of the onset of atrioventricular block, and plasma concentrations of compound (I) and siponimod. The plasma was collected by centrifuging the blood and cryopreserved at about -20°C. After thawing of the plasma, plasma drug concentrations were measured using LC/MS/MS after deproteinization with acetonitrile.

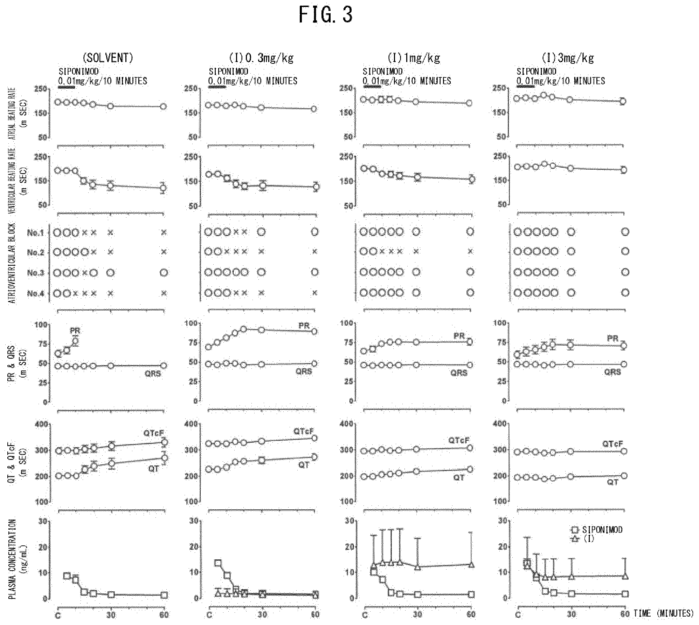

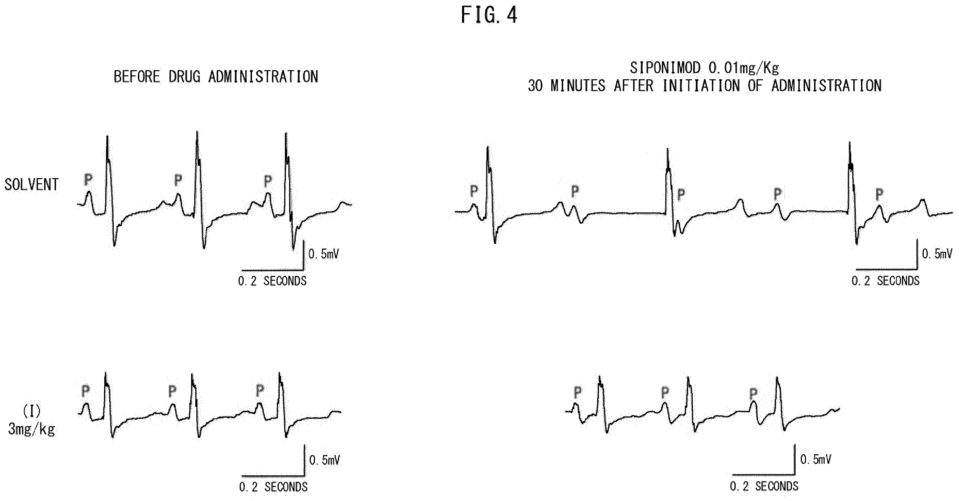

FIG. 3 shows changes over time in parameter measurements in each group, and FIG. 4 shows the resultant representative electrocardiographic waveforms. In FIG. 3, the measurements are shown as mean ± standard error. Abbreviations, etc. are as follows.
(I): compound (I)
Atrioventricular block: the presence or absence of atrioventricular block
(× denotes second- or third-degree atrioventricular block)
PR & QRS: atrioventricular conduction time and ventricular activation time
QT & QTcF: ventricular repolarization time and heart rate-corrected ventricular repolarization time
Plasma concentration: the plasma concentration of siponimod or compound (I)

FIG. 3 shows comparison of the effects of no administration of compound (I) and administration of compound (I) at doses of 0.3 mg/kg, 1 mg/kg, and 3 mg/kg. Regarding the ventricular beating rate, noticeable siponimod-induced bradycardia was observed in the case of no administration. In contrast, in the compound (I) administration group, bradycardia was improved in a dose-dependent manner. In particular, bradycardia was completely restored in the 3 mg/kg administration group.

Regarding the presence or absence of the onset of atrioventricular block, siponimod-induced atrioventricular block was observed in many cases (three out of four cases) in the non-administration group. In contrast, atrioventricular block was not observed in two cases (50%) in the 0.3 mg/kg administration group of compound (I) and in three cases (75%) in the 1 mg/kg administration group, and atrioventricular block was suppressed in all four cases (100%) in the 3 mg/kg administration group. Similarly, siponimod-induced prolongation of QT time was almost completely suppressed.

FIG. 4 shows typical electrocardiograms in the compound (I) non-administration group and the 3 mg/kg administration group. In the non-administration group, about a half reduction in heat rate was observed; i.e., noticeable bradycardia was observed. In contrast, in the 3 mg/kg administration group, such siponimod-induced bradycardia was completely prevented.

As shown in FIG. 3 and FIG. 4, compound (I) prevented the onset of siponimod-induced bradyarrhythmia (atrioventricular block) in a dose-dependent manner, and prevented a reduction in heart rate.

### Evaluation Example 3

### Termination Effect of Compound (I) on Fingolimod-Induced Bradyarrhythmia in Isoflurane-Anesthetized Guinea Pig Model

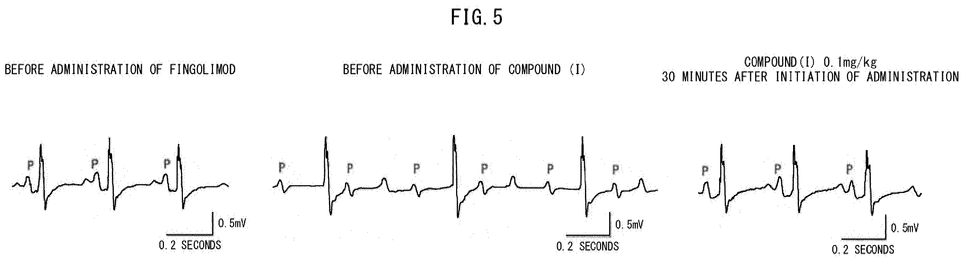

Hartley guinea pigs (7 to 10 weeks old, male, available from Japan SLC, Inc.) were subjected to anesthetic induction by inhalation of isoflurane (available from Pfizer Japan Inc.), and each animal was held on an operating table having a heater function. The trachea was incised, and a tracheal cannula (KN-375, available from Natsume Seisakusho Co., Ltd.) was inserted. The tracheal cannula was connected to a ventilator (SN-480-7, available from Shinano Seisakusho Co., Ltd.), and artificial respiration was controlled by supplying 100% oxygen at a volume of 10 mL and a ventilation rate of 60 times/minute. Isoflurane (available from Pfizer Japan Inc.) anesthesia (about 1 to 4%, carrier gas: 100% oxygen) was used for maintenance of anesthesia. A catheter (BD intramedic polyethylene tubing PE50, available from Becton, Dickinson and Company) charged with 50 units/mL heparin (available from AY PHARMACEUTICALS CO., LTD.) was inserted into each of the carotid artery and the jugular vein and placed in the blood vessel. Systemic blood pressure was measured with a high-sensitivity DC amplifier (AD-611J, available from NIHON KOHDEN CORPORATION) via the catheter placed in the carotid artery. Electrodes were attached to the body surface, and a body surface electrocardiogram was measured with a high-sensitivity amplifier (AB-611J, available from NIHON KOHDEN CORPORATION). Blood pressure and electrocardiogram data were acquired and analyzed with PowerLab/Labchart system (available from ADInstruments). After stabilization of each parameter, fingolimod hydrochloride (available from Nissan Chemical Corporation) was continuously administered intravenously at 0.1 mg/kg for 10 minutes via the catheter placed in the jugular vein, followed by observation of the presence or absence of atrioventricular block. After confirmation that atrioventricular block continued for 10 minutes from the onset thereof, a solvent or compound (I) dihydrochloride was continuously administered intravenously at 0.1 mg/kg for 30 minutes, and the effect of terminating atrioventricular block was observed during the intravenous administration. The solvent used was saline (available from Otsuka Pharmaceutical Factory, Inc.). Data were recorded and blood was collected 10, 20, and 30 minutes after initiation of administration of compound (I) dihydrochloride and at the time of termination of atrioventricular block. The observation was terminated 30 minutes after initiation of the administration, and the animal was euthanized by exsanguination or intravenous administration of a large amount of saturated potassium chloride solution under isoflurane anesthesia. Data were analyzed for the presence or absence of the termination, the time required for the termination, and the plasma concentration of compound (I) at the time of termination. The plasma was collected by centrifuging the blood and cryopreserved at about -20°C. After thawing of the plasma, plasma drug concentrations were measured using LC/MS/MS after deproteinization with acetonitrile. FIG. 5 shows the resultant representative electrocardiographic waveforms.

Table 1 shows the number of cases where atrioventricular block was terminated, the time required for the termination of atrioventricular block, and the compound (I) concentration at the time of termination. In Table 1, the measurements are shown as mean ± standard error.

**Table 1**

| Group | Number of cases where atrioventricular block was terminated | Time required for the termination of atrioventricular block (seconds) | Compound (I) concentration at the time of termination (ng/mL) |
|---|---|---|---|
| Solvent | 0/5 | - | - |
| Compound (I) | 5/5 | 750 ± 43 | 30.9 ± 1.9 |

As shown in Table 1, compound (I) completely terminated bradyarrhythmia (atrioventricular block: A-V block) induced by administration of fingolimod. FIG. 5 shows typical electrocardiograms before administration of fingolimod, before administration of compound (I), and 30 minutes after initiation of administration of 0.1 mg/kg of compound (I) dihydrochloride. About a half reduction in heat rate; i.e., noticeable bradycardia was observed before administration of compound (I). In contrast, such fingolimod-induced bradycardia was completely recovered 30 minutes after initiation of administration of 0.1 mg/kg of compound (I) dihydrochloride.

### Evaluation Example 4

### Termination Effect of Compound (I) on Siponimod-Induced Bradyarrhythmia in Isoflurane-Anesthetized Guinea Pig Model

Hartley guinea pigs (7 to 10 weeks old, male, available from Japan SLC, Inc.) were subjected to anesthetic induction by inhalation of isoflurane (available from Pfizer Japan Inc.), and each animal was held on an operating table having a heater function. The trachea was incised, and a tracheal cannula (KN-375, available from Natsume Seisakusho Co., Ltd.) was inserted. The tracheal cannula was connected to a ventilator (SN-480-7, available from Shinano Seisakusho Co., Ltd.), and artificial respiration was controlled by supplying 100% oxygen at a volume of 10 mL and a ventilation rate of 60 times/minute. Isoflurane (available from Pfizer Japan Inc.) anesthesia (about 1 to 4%, carrier gas: 100% oxygen) was used for maintenance of anesthesia. A catheter (BD intramedic polyethylene tubing PE50, available from Becton, Dickinson and Company) charged with 50 units/mL heparin (available from AY PHARMACEUTICALS CO., LTD.) was inserted into each of the carotid artery and the jugular vein and placed in the blood vessel. Systemic blood pressure was measured with a high-sensitivity DC amplifier (AD-611J, available from NIHON KOHDEN CORPORATION) via the catheter placed in the carotid artery. Electrodes were attached to the body surface, and a body surface electrocardiogram was measured with a high-sensitivity amplifier (AB-611J, available from NIHON KOHDEN CORPORATION). Blood pressure and electrocardiogram data were acquired and analyzed with PowerLab/Labchart system (available from ADInstruments). After stabilization of each parameter, siponimod (available from Nissan Chemical Corporation) was continuously administered intravenously at 0.01 mg/kg for 10 minutes via the catheter placed in the jugular vein, followed by observation of the presence or absence of atrioventricular block. The solvent used was a solution prepared by mixing of Polyethylene Glycol 400 (available from KANTO CHEMICAL CO., INC.) and dimethyl sulfoxide (available from FUJIFILM Wako Pure Chemical Corporation) at 9 : 1 (volume ratio). After confirmation that atrioventricular block continued for 10 minutes from the onset thereof, a solvent or compound (I) dihydrochloride was continuously administered intravenously at 0.1 mg/kg for 30 minutes, and the effect of terminating atrioventricular block was observed during the intravenous administration. The solvent used was saline (available from Otsuka Pharmaceutical Factory, Inc.). Data were recorded and blood was collected 10, 20, and 30 minutes after initiation of administration of compound (I) dihydrochloride and at the time of termination of atrioventricular block. The observation was terminated 30 minutes after initiation of the administration, and the animal was euthanized by exsanguination or intravenous administration of a large amount of saturated potassium chloride solution under isoflurane anesthesia. Data were analyzed for the presence or absence of the termination, the time required for the termination, and the plasma concentration of compound (I) at the time of termination. The plasma was collected by centrifuging the blood and cryopreserved at about -20°C. After thawing of the plasma, plasma drug concentrations were measured using LC/MS/MS after deproteinization with acetonitrile.

Table 2 shows the number of cases where atrioventricular block was terminated, the time required for the termination of atrioventricular block, and the compound (I) concentration at the time of termination. In Table 2, the measurements are shown as mean ± standard error.

**Table 2**

| Group I | Number of cases where atrioventricular block was terminated | Time required for the termination of atrioventricular block (seconds) | Compound (I) concentration at the time of termination (ng/mL) |
|---|---|---|---|
| Solvent | 1/5 | 637 | - |
| Compound (I) | 5/5 | 638 ± 75 | 22.7 ± 3.1 |

As shown in Table 2, compound (I) completely terminated bradyarrhythmia (atrioventricular block) induced by administration of siponimod.

### Evaluation Example 5

### Therapeutic Effect of Compound (I) on Siponimod-Induced Bradyarrhythmia in Human iPS Cell-Derived Atrial Myocytes

Cryopreserved human iPS cell-derived atrial myocytes (Human iPSC-derived atrial cardiomyocytes, available from Axol Bioscience) were thawed and seeded at 1,400,000 cells/well on a fibronectin-coated 6-well plate, followed by culturing in an incubator at 37°C and 5% CO₂ for 11 days. During the culture, the entire amount of medium was changed every other day. After the elapse of 11 days, human iPS cell-derived atrial myocytes that started beating were removed from the plate and recovered with TrypLE Select Enzyme (available from ThermoFisher Scientific). The atrial myocytes were seeded at 25,000 cells/2 µL on a fibronectin-coated 6-well MEA Chip (60-6well MEA200/30iR-Ti-tcr, available from Multi Channel Systems) and cultured in an incubator at 37°C and 5% CO₂ for one hour. Thereafter, 400 µL of medium was added and cultured in an incubator at 37°C and 5% CO₂ for 13 days. During the culture, a half amount of the medium was changed every other day. After the elapse of eight days, the MEA Chip was set in a multi-electrode array system (MEA2100, available from Multi Channel Systems) to thereby obtain extracellular potentials under spontaneous beating. After stabilization of the beating rate, 0.1 µM of siponimod was added, and the effect of siponimod on the beating rate was observed for 10 minutes. Thereafter, 0.1 µM of compound (I) or a solvent (dimethyl sulfoxide (available from FUJIFILM Wako Pure Chemical Corporation)) was further added, and the effect thereof on the beating rate was observed for 10 minutes. Data were analyzed with Labchart System (available from ADInstruments).

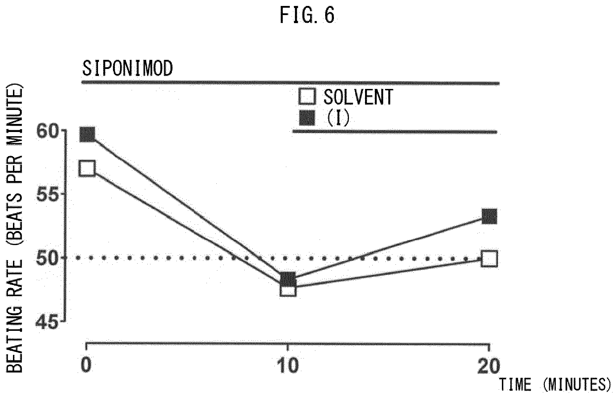

FIG. 6 shows the effect of addition of siponimod on beating rate and the effect of compound (I) on an improvement in beating rate. In FIG. 6, the measurements are shown as mean ± standard error, and (I) denotes compound (I).

As shown in FIG. 6, the addition of siponimod caused bradyarrhythmia (sinus bradycardia) with a beating rate of 50 beats/minute or less, and the addition of compound (I) increased the beating rate and improved bradyarrhythmia.

### Evaluation Example 6

### Examination of Effect of Compound (I) on Fingolimod-Induced Decrease in Blood Lymphocytes in Guinea Pig

Hartley guinea pigs (8 weeks old, male, available from Japan SLC, Inc.) were fasted for about 16 hours, and then a solvent or compound (I) (free form) was administered by oral gavage to each animal at a dose of 10 or 30 mg/kg (volume: 5 mL/ kg). The solvent used was 0.5 w/v% methyl cellulose 400 solution (available from FUJIFILM Wako Pure Chemical Corporation). Thirty minutes after the administration, a solvent or fingolimod hydrochloride was administered by oral gavage to the animal at a dose of 0.1 mg/kg (volume: 5 mL/ kg). The solvent used was saline (available from Otsuka Pharmaceutical Factory, Inc.). Blood was collected from the jugular vein, the subclavian vein, or the abdominal vena cava before administration of compound (I) and 5 and 24 hours after administration of fingolimod hydrochloride. Blood cell components were analyzed with a multi-item automatic blood cell counter (XN-1000, available from Sysmex Corporation). The plasma was collected by centrifuging the blood and cryopreserved at about -20°C. After thawing of the plasma, plasma drug concentrations were measured using LC/MS/MS after deproteinization with acetonitrile.

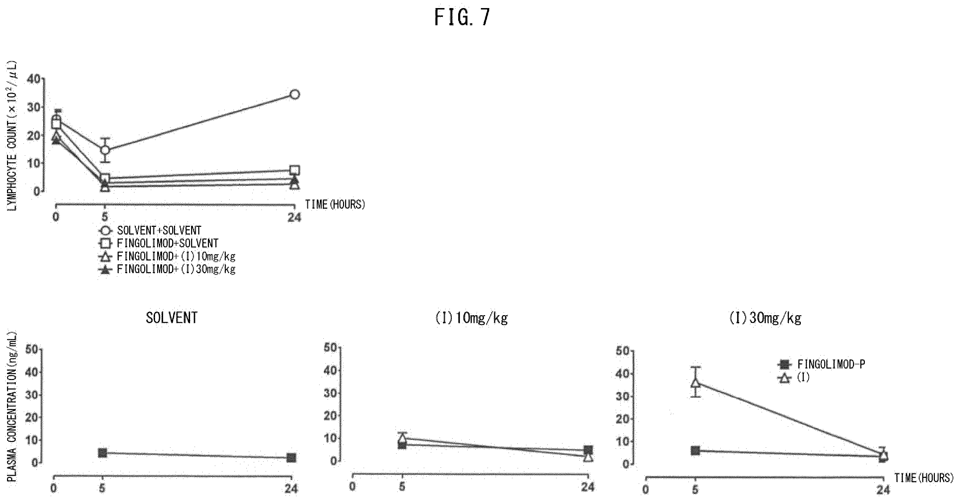

FIG. 7 shows the effects of fingolimod and compound (I) on peripheral blood lymphocyte counts in guinea pigs. In FIG. 7, the measurements are shown as mean ± standard error, and (I) denotes compound (I).

As shown in FIG. 7, the administration of fingolimod decreased the number of peripheral blood lymphocytes in guinea pigs, and this action was also shown in the presence of compound (I). The results indicated that compound (I) does not inhibit the peripheral blood lymphocyte decreasing action (i.e., main drug efficacy) of fingolimod.

### Evaluation Example 7

### Therapeutic Effect of Compound (I) on Propranolol-Induced Bradyarrhythmia in Conscious Rat with Implanted Telemetry Transmitter for Measuring Heart Rate

Wistar rats (10 weeks old, male, available from Kiwa Laboratory Animals Co., Ltd.) were anesthetized by inhalation of Sevofrane (available from Maruishi Pharmaceutical Co., Ltd.) and held on an operating table. An implantable telemetry transmitter ETA-F10 (DSI) was placed subcutaneously in each rat, and one lead was fixed to each of the upper right chest and the left lower chest. After being fully awakened, the rat was placed in a cage on the receiving board, and a body surface electrocardiogram was measured with a high-sensitivity amplifier. Electrocardiogram data were acquired and analyzed with LabChart Pro & LabChart module (available from Bio Research Center Co., Ltd.). After stabilization of the heart rate, propranolol (Inderal Injection, available from AstraZeneca K.K.) was administered by intraperitoneal injection at a dose of 1 mg/kg. After the elapse of about 30 minutes, the stabilization of the heart rate was determined, and then saline or compound (I) dihydrochloride was administered by intraperitoneal injection at a dose of 1 mg/kg. Thirty minutes after the administration, the heart rate in a stable state was measured for evaluation. The solvent used was saline (available from Otsuka Pharmaceutical Factory, Inc.). After completion of the observation, the telemetry transmitter was removed from the animal under sevoflurane anesthesia, and the animal was euthanized by exsanguination or sevoflurane overdose.

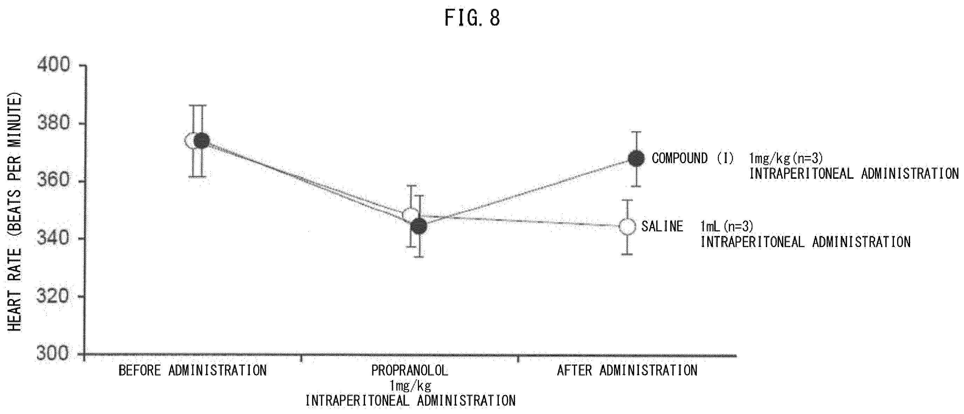

FIG. 8 shows changes in heart rate as determined before and after administration of compound (I) or saline with respect to propranolol-induced bradyarrhythmia.

As shown in FIG. 8, compound (I) improved bradycardia induced by administration of propranolol.

### Evaluation Example 8

### Therapeutic Effect of Compound (I) on Propranolol-Induced Bradycardia in Conscious Beagle Dog

A peripheral venous path was secured with an 18G needle in the right forelimb of a beagle dog (body weight: about 10 kg, female, available from Oriental Yeast Co., Ltd.), and then held on an operating table. Heart rate was monitored with an electrocardiographic monitor worn on the animal. A manchette was worn on the left forelimb, and blood pressure was measured with an Omron automatic electronic sphygmomanometer. The left ventricular function was measured by the M-mode method using the echocardiographic left parasternal short-axis two-chamber view. After stabilization of the heart rate, propranolol (Inderal Injection, available from AstraZeneca K.K.) was administered by bolus intravenous injection at a dose of 2 mg/kg, followed by continuous intravenous injection at a dose of 1 mg/minute with a syringe pump. After the elapse of about 30 minutes, the stabilization of the heart rate was determined, and then saline and compound (I) dihydrochloride (0.01 mg/kg, 0.1 mg/kg, 1 mg/kg) were administered by bolus intravenous injection at 10-minute intervals. About 10 minutes after each administration, heart rate, blood pressure, and left ventricular function were measured and evaluated in a stable state. The solvent used was saline (available from Otsuka Pharmaceutical Factory, Inc.).

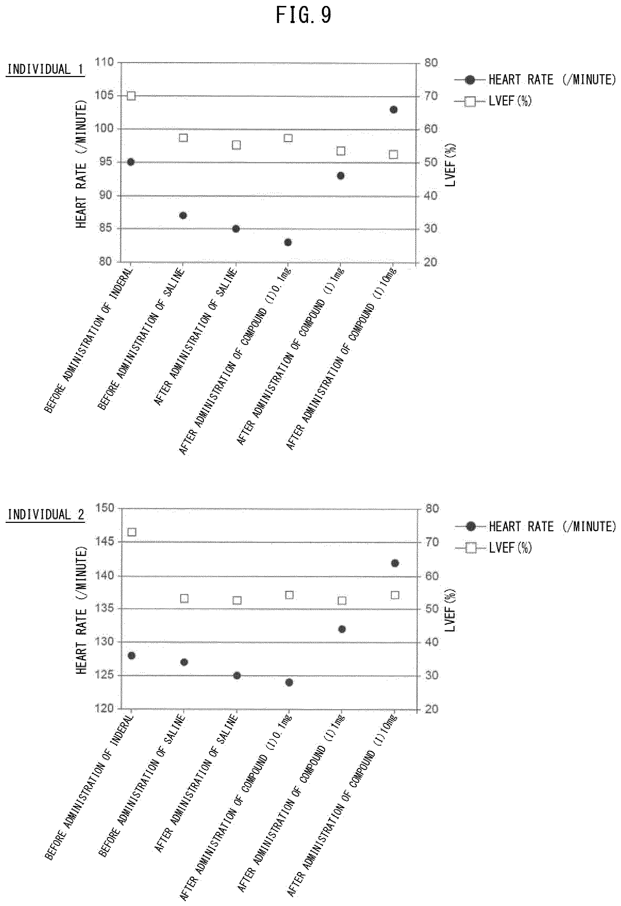

FIG. 9 shows changes in heart rate and left ventricular ejection fraction (LVEF) as determined before and after administration of saline and compound (I) with respect to propranolol-induced bradycardia.

As shown in FIG. 9, compound (I) improved bradycardia induced by administration of propranolol. Compound (I) did not affect the cardiac contraction inhibitory action by administration of propranolol.

### Evaluation Example 9

Therapeutic Effect of Compound (I) on Verapamil-Induced Bradycardia in Conscious Beagle Dog

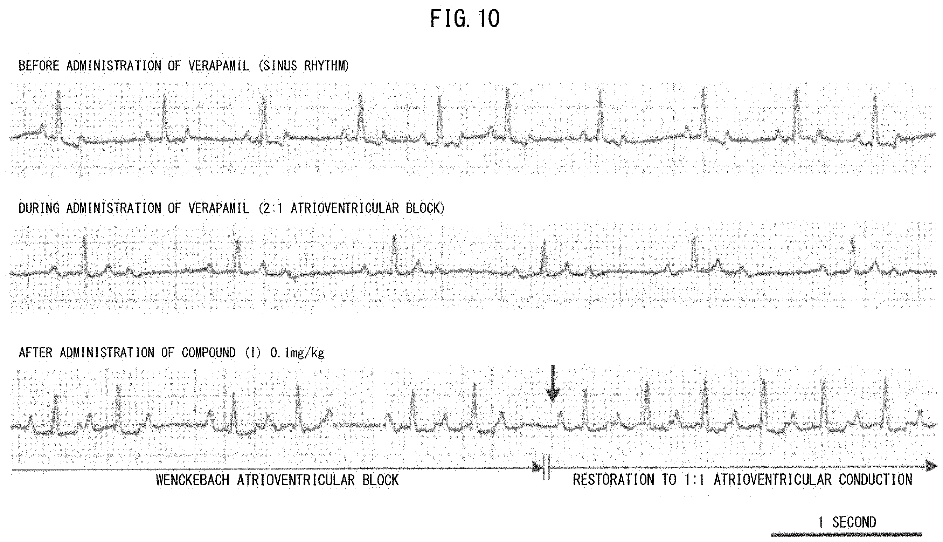

A peripheral venous path was secured with an 18G needle in the right forelimb of a beagle dog (body weight: about 10 kg, female, available from Oriental Yeast Co., Ltd.), and then held on an operating table. Heart rate was monitored with an electrocardiographic monitor worn on the animal. A manchette was worn on the left forelimb, and blood pressure was measured with an Omron automatic electronic sphygmomanometer. The left ventricular function was measured by the M-mode method using the echocardiographic left parasternal short-axis two-chamber view. After stabilization of the heart rate, verapamil (intravenous injection of Vasolan, available from Eisai Co., Ltd.) was administered by continuous intravenous injection at a dose of 0.8 mg/kg/hour with a syringe pump. About 30 minutes after the initiation of administration, the dose was increased to 2.4 mg/kg/hour, and continuous infusion was continued. Since 2:1 atrioventricular block was continuously determined 17 minutes after an increase in the dose, the administration of verapamil was discontinued. Subsequently, saline and compound (I) dihydrochloride (0.01 mg/kg, 0.1 mg/kg, 1 mg/kg) were administered by bolus intravenous injection at 10-minute intervals. About 10 minutes after each administration, electrocardiogram, heart rate, blood pressure, and left ventricular function were measured and evaluated in a stable state. The solvent used was saline (available from Otsuka Pharmaceutical Factory, Inc.). FIG. 10 shows changes in heart rate as determined before and after administration of compound (I) or saline with respect to verapamil-induced bradycardia. FIG. 10 also shows a representative electrocardiographic waveform as obtained when 0.1 mg/kg of compound (I) exhibited a termination effect on verapamil-induced bradyarrhythmia (2:1 atrioventricular block).

As shown in FIG. 10, compound (I) improved bradyarrhythmia (2:1 atrioventricular block) induced by administration of verapamil.

### INDUSTRIAL APPLICABILITY

According to the present invention, compound (I) of the present invention can be used as a therapeutic agent for drug-induced bradycardia and bradyarrhythmia.

## Claims

1. A therapeutic agent for drug-induced bradycardia and bradyarrhythmia comprising, as an active ingredient, the following compound (I): 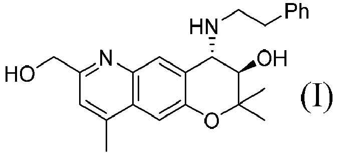 (wherein Ph is a phenyl group) or a pharmacologically acceptable salt of the compound.

2. The therapeutic agent according to claim 1, wherein the drug-induced bradyarrhythmia is bradyarrhythmia induced by an S1P receptor modulator.

3. The therapeutic agent according to claim 1, wherein the drug-induced bradyarrhythmia is bradyarrhythmia induced by an S1P1 modulator.

4. The therapeutic agent according to claim 1, wherein the drug-induced bradyarrhythmia is bradyarrhythmia induced by fingolimod.

5. The therapeutic agent according to claim 1, wherein the drug-induced bradyarrhythmia is bradyarrhythmia induced by siponimod.

6. The therapeutic agent according to claim 1, wherein the drug-induced bradyarrhythmia is bradyarrhythmia induced by a β-blocker.

7. The therapeutic agent according to claim 1, wherein the drug-induced bradyarrhythmia is bradyarrhythmia induced by propranolol.

8. The therapeutic agent according to claim 1, wherein the drug-induced bradyarrhythmia is any of the following bradycardias: (a) sinus bradycardia, (b) sinus arrest, (c) sinoatrial block, (d) atrioventricular block, (e) sinus node dysfunction, (f) bradycardic heart failure, and (g) bradycardic atrial fibrillation.

9. The therapeutic agent according to claim 1, wherein the drug-induced bradyarrhythmia is sinus bradycardia or atrioventricular block.

10. The therapeutic agent according to claim 1, wherein the drug-induced bradyarrhythmia is sinus bradycardia.

11. The therapeutic agent according to claim 1, wherein the drug-induced bradyarrhythmia is atrioventricular block.

12. The therapeutic agent according to claim 1, wherein the therapeutic agent is for the purpose of prevention.

13. The therapeutic agent according to claim 1, wherein the therapeutic agent is for the purpose of termination.

14. A therapeutic agent for drug-induced bradycardia and bradyarrhythmia comprising, as an active ingredient, a compound having a KACh channel inhibitory action or a pharmacologically acceptable salt of the compound.

15. The therapeutic agent according to any one of claims 1 to 14, wherein the therapeutic agent for drug-induced bradycardia and bradyarrhythmia is a therapeutic agent for drug-induced bradyarrhythmia.

16. The therapeutic agent according to any one of claims 1 to 14, wherein the therapeutic agent for drug-induced bradycardia and bradyarrhythmia is a therapeutic agent for bradyarrhythmia.
